# EUROPEAN PATENT SPECIFICATION

(11) **EP 2 762 055 B1**
(45) Date of publication and mention of the grant of the patent: **27.04.2016**
(21) Application number: 13000552.3
(22) Date of filing: 04.02.2013
(51) Int. Cl.: A61B 1/00, A61B 1/227, A61B 1/05

(54) **Otoscope**
Otoskop
Otoscope

(43) Date of publication of application: 06.08.2014
(73) Proprietor: Helen of Troy Limited, St. Michael (BB)
(72) Inventor: Ruppersberg, J. Peter, 1807 Blonay (CH); Lepple-Wienhues, Albrecht, 25300 Pontarlier (FR)
(74) Representative: Graf von Stosch, Andreas

(56) References cited:
- EP-A1- 2 289 391
- EP-A2- 2 277 439
- TW-A- 201 225 896
- US-A- 5 919 130
- US-A1- 2011 063 428
- US-A1- 2013 027 515
- MARTIN WILKE ET AL: "Prospects and limits in wafer-level-packaging of image sensors", ELECTRONIC COMPONENTS AND TECHNOLOGY CONFERENCE (ECTC), 2011 IEEE 61ST, IEEE, 31 May 2011 (2011-05-31), pages 1901-1907, XP031996800, DOI: 10.1109/ECTC.2011.5898775 ISBN: 978-1-61284-497-8
- Martin Wäny ET AL: "Ultra small digital image sensor for endoscopic applications", Proc. of 2009 International Image Sensor Workshop, 26 March 2009 (2009-03-26), XP055035306, Bergen, Norway Retrieved from the Internet: URL:http://www.imagesensors.org/Past%20Wor kshops/2009%20Workshop/2009%20Papers/044_p aper_waeny_awaiba_endoscopy.pdf [retrieved on 2012-08-10]

## Description

The invention refers to an otoscope comprising a handle portion allowing a user to manipulate the otoscope during its application, and further comprising a head portion exhibiting a substantially tapering form extending along a longitudinal axis of the head portion, wherein the head portion has a proximal end adjacent to the handle portion and a smaller distal end configured to be introduced in an ear canal of a patient's outer ear.

An otoscope (sometimes also called "auriscope") is a medical device which is used to look into ears. The corresponding method of doing so is called "otoscopy". Otoscopy is a standard medical examination technique established more than 100 years ago. Medical students learn otoscopy early in their studies during the practical course in physiology. Typical diagnoses based on otoscopic examination are: otitis media, middle ear hydros, otitis externa, and eardrum perforation. However, otoscopy is also used to generally identify and observe object's in the ear, such as earwax, hair and the eardrum.

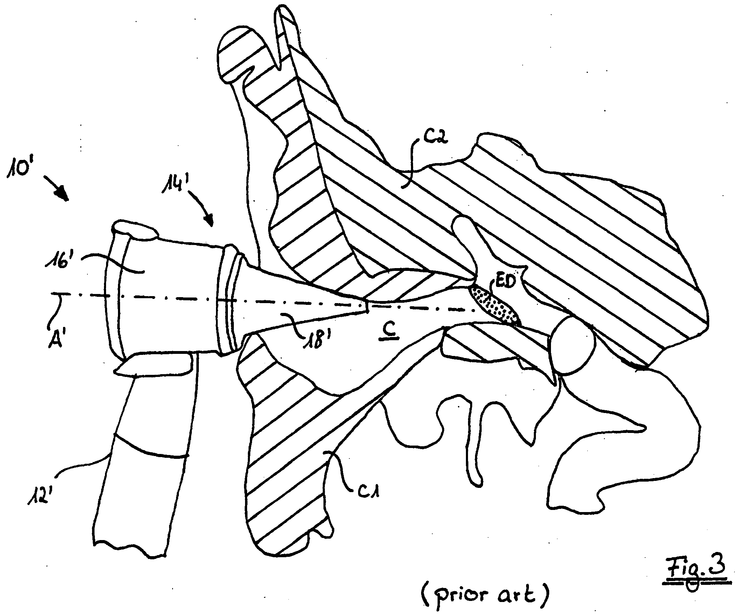

A typical otoscope 10' as used for decades in otoscopy is shown in figure 3. The otoscope 10' comprises a handle portion 12' allowing the user to manipulate the otoscope during its application. The term "to manipulate" in this context refers to different kinds of manipulation, such as - but not limited to - holding the otoscope, aligning the otoscope with respect to the patient's ear, and turning on or off a light. The otoscope 10' further comprises a head portion 14' connected to the handle portion 12'. The head portion 14' exhibits a substantially tapering form - usually a conical form - extending along a longitudinal axis A' of the head portion 14'. The head portion 14' is substantially comprised of an empty funnel, wherein the tip of the funnel typically has a diameter of 3 millimeters. Furthermore, the head portion 14' has a proximal end 16' adjacent to the handle portion 12' and a smaller distal end 18' configured to be introduced in an ear canal C of a patient's outer ear. The term "end" in this context does not mean a single point but rather refers to a region or section of the head portion 14', wherein the proximal end 16' is located opposite to the distal end 18' with respect to the longitudinal axis A'. The ear canal C is partly surrounded by soft connective tissue C1 and - further down towards the middle ear - partly by hard bone C2.

The working principle of the known otoscope is typically to observe and simultaneously illuminate the patient's eardrum ED through the empty funnel with the 3mm tip pushed deeply into the ear canal C. Normally, the eardrum ED is not visible from outside the ear, due to the natural curvature of the ear canal C. In order to overcome the natural curvature of the ear canal C, the skilled physician has to carefully pull the outer ear upward and to the back while carefully pushing the tip of the funnel as deeply as necessary to observe the eardrum. The ear canal C has to be deformed in such a way that the physician has a free view onto the eardrum ED along the optical axis of the otoscope 10', wherein the optical axis corresponds to the longitudinal axis A' of the head portion 14'. The optics of an otoscope is situated only at the wider end of the funnel at its proximal end 16' and essentially consists of a lamp and a lens (not shown) to magnify the image of the eardrum ED.

The otoscopy procedure needs manual skills and significant training to make it possible to carefully push the funnel into the ear canal C while looking inside and manipulating the curvature of the ear canal C by pulling the ear. For example, it is very important for the trained physician to brace the hand holding the otoscope against the patient's head to avoid injury to the ear canal C by placing the index finger or little finger against the head. In particular in young children - where the inner part of the ear canal is relatively short and sudden head movement during the examination may occur - there is a risk of penetration of the very sensitive ear canal skin or even of the eardrum ED. Besides pain and handicapped hearing, such an injury may even induce cardiovascular complications through a vagal overstimulation and therefore has to be avoided by all means.

Furthermore, especially in an inflamed ear, the mechanical manipulation of "straightening" the ear canal C typically causes considerable discomfort or even pain, rendering the examination of an infant even more difficult.

For the above reasons, reliably and securely handling an otoscope of the art is currently subject to only well trained physicians and not amenable to the larger community of practitioners. A study recently published in the US as a result of a survey has shown that even physicians often fail to (correctly) determine the status of e.g. the subject's eardrum or fail to correctly interpret the image provided by the otoscope (i.e. correct and meaningful object recognition). Such failures result in misinterpretation of the status of the inner ear canal or the eardrum. As a consequence, e.g. over-medication with antibiotics for treating supposed inflammations of the eardrum occurs, because physicians tend to err on the side of caution, or meaningless image interpretation occurs.

Notably, there also exist other otoscopic devices, as e.g. video otoscope, allowing a skilled expert to capture images of the subject's eardrum and the ear canal. Such video otoscopes comprise a bundle of light guides extending from the distal end of the head portion to a CCD-chip located remote from the distal end. The achievable resolution of the images depends on the number of light guides. In order to obtain images having a satisfying resolution, a significant number of individual light guides must be provided rendering devices by far too expensive for routine care. Moreover, all of the known video otoscopes having the CCD-chip located remote from the distal end of the head portion require superior handling-skills by he physician. For the above reasons, they are not configured and suitable for domestic use by a larger community of practitioners, nor use by laypersons.

All otoscopes currently on the market - including video otoscopes - generally are based on the following fundamental design: a relatively thin open funnel. Length, angle, field of vision and size of the funnels are essentially similar for all marketed otoscopes. As a result of these common characteristics, ease of use (due to safety issues) is limited for such devices. Methods for reliable detection of objects in the ear canal, including the eardrum, are remarkably intricate with such known otoscopes.

EP 2 289 391 A1 describes an otoscope according to the preamble of claim 1, with a removable head portion and a fastening ring, which, when loosened, allows the displacement of the head portion.

Consequently, until today otoscopy has almost been exclusively applied by medical doctors. And even among medical doctors, only a minor percentage is sufficiently trained to carry out otoscopy in a reliable and appropriate way. However, since otitis media is the most frequent disease causing high fever in young children, and to exclude otitis media is a major reason for seeing a pediatrician, there is an urgent need for a parental check of the ear. Parents may also benefit from an otoscope that can be securely used by laypersons at home in order to check whether an ear canal of their child is blocked by massive earwax and/or foreign objects.

It is therefore an object of the present invention to provide an otoscope that allows for domestic application by laypersons and medical doctors without extensive otoscopy training and without any - or at least with a significantly reduced - risk of causing injuries to the patient.

This object is achieved according to the present invention by an otoscope exhibiting the features of claim 1. Preferred embodiments represent the subject-matter of the dependent claims.

The present disclosure relates to an otoscope of the generic type as described above, wherein the otoscope further comprises an electronic imaging unit positioned at the distal end of the head portion.

Providing a small electronic imaging unit at the distal end of the head portion allows to "see" the patient's eardrum without the need to deform the patient's ear canal, or at least without having to deform the ear canal to such an extent as with the above described conventional otoscope. The reason for this is that there is no need for the "viewing direction" (optical axis) of the electronic imaging unit to correspond to the longitudinal axis of the head portion of the otoscope. Instead, the optical axis of the electronic imaging unit may be arranged at an angle with respect to the longitudinal axis, allowing the device to "look around the corner". An additional or alternative reason is that the field of vision of an electronic imaging unit provided at the distal end of the head portion can be much greater than the field of vision achievable with the relatively acute empty funnel of the otoscope according to the prior art.

Furthermore, the distal end of the head portion of the otoscope according to the present disclosure does not need to have a conical shape with a relatively thin open funnel, which shape bears the risk of introducing the distal end of the head portion too far into the ear canal, so as to cause serious injuries to the patient. Instead, the outer shape of the distal end of the head portion can be designed in such a way that it is practically impossible to introduce it too far into the ear canal. Thus, the otoscope according to the present disclosure can be securely and reliably operated even by laypersons without the risk of causing injuries to the patient.

The otoscope according to the present disclosure may comprise further features that are provided, for example, by modern digital photo cameras. For example, the otoscope may comprise visual output means, such as a display, and/or acoustic output means, such as a loudspeaker, and/or a storage card slot for inserting a storage card to store the acquired images, and/or a cable connection port, such as an USB-port, and/or a wireless connection, such as Bluetooth®, WIFI®, and/or an energy supply, such as a battery.

The electronic imaging unit may be a video camera, preferably a wide angle color video camera. The term "wide angle" in this context refers to angels of at least 80°, preferably of at least 110°, e.g. 120°. Such wide angle cameras allow detection of the patient's eardrum, even if the optical axis ("viewing direction") of the camera is not directly centered to the eardrum and even if the eardrum is relatively remote from the camera, compared to the distance between the eardrum and the tip end of a conventional otoscope head during application. Using a color video camera is advantageous, allowing determination of the color of the eardrum and/or of the inner portion of the ear canal. Thus, inflammations can be detected by the degree of reddishness.

The electronic imaging unit may be a wafer-level camera of a substantially flat configuration, having dimensions of less than 3mm x 3mm, preferably less than 2mm x 2mm, even more preferable of about 1 mm x 1 mm or even less than 1 mm x 1mm. Wafer-level cameras refer to a relatively new technology. They can be produced small in size with only about 3 microns per pixel. Therefore, wafer-level imaging technology allows obtaining images of "sufficient" resolution of the eardrum, e.g. images of 250 pixels x 250 pixels, with a footprint of the camera including lens of only about 1 mm x 1 mm or even smaller.

The electronic imaging unit may be positioned substantially centrically with respect to the longitudinal axis of the head portion. If the electronic imaging unit is positioned on the longitudinal axis of the head portion, the substantially flat electronic imaging unit is preferable inclined with respect of the longitudinal axis of the head portion, so that the optical axis (or the "main viewing direction") of the electronic imaging unit is angled with respect to the longitudinal axis of the head portion, allowing the otoscope to "look around the corner". Consequently, the otoscope according to the present disclosure does not have to bee introduced as deeply into the patient's ear compared to a conventional otoscope of the prior art.

Alternatively, the electronic imaging unit may be positioned radially offset from the longitudinal axis of the head portion. Such a configuration also allows obtaining a free view onto the eardrum without having to introduce the electronic imaging unit as deeply as it would be necessary if the electronic imaging unit were placed just centrally on the longitudinal axis of the head portion. The offset may be at least 1 mm, preferably at least 2mm, more preferably at least 3mm from the longitudinal axis.

The head portion is preferably shaped in such a way that its distal end comprising the electronic imaging unit can be introduced only as deep into the ear canal as not to touch the eardrum. The ear canal of the patient's outer ear is limited by the eardrum. Notably, the ear canal of the patient's outer ear comprises an outer part which refers to a portion of the patient's outer ear (i.e. the patient's external auditory canal) that is surrounded by soft connective tissue and that usually comprises hair and earwax. The outer part comprises approximately the outer half of the ear canal of the patient's outer ear. Furthermore, the ear canal of the patient's outer ear also comprises an inner part which refers to a portion of the patient's outer ear (i.e. the patient's external auditory canal) that is surrounded by hard skull bone and that is usually free from any hair and earwax. This portion extends from the proximal end the outer part of the ear canal of the patient's outer ear to the eardrum. The inner part of the ear canal is very sensitive to pain in case of injury by mechanical friction. Injuring the inner part of the ear canal even bears the risk of cardiovascular complications through vagal overstimulation.

Preferably, a tip portion of the distal end can be introduced into the ear canal of the patient's outer ear no further than to a distance from the eardrum of at least a few millimeters, preferably of at least 3mm.

As already mentioned above, the tapering head portion of the otoscope according to the present disclosure can be shaped with a blunt, rounded tip end, as compared to a conventionally known otoscope, thereby reducing the risk of introducing injury or discomfort to the patient. Thus, the device can be securely handled by laypersons. The otoscope according to the present invention, nevertheless, allows detecting the eardrum, since the electronic imaging unit is provided at the distal end of the head portion.

Preferably, the distal end of the head portion is provided with a round and smooth shape. Moreover, the distal end may be made from a relatively soft material, such as silicone, or it may comprise an outer surface made of such a soft material. Furthermore, the longitudinal force upon introduction into the ear canal can be limited by a telescoping mechanism or the use of an elastic element.

The functional concept of a conventional otoscope as described above, however, requires the tip end of the head portion to be relatively small and acute (sharp), usually having a diameter of only about 3mm. It is noted that the diameter of the inner part of the outer ear canal of an adult is about 4mm. Therefore, if the user (untrained) does not pay attention, the tip portion might be introduced deeply into the inner part of the outer ear canal causing serious injuries to the patient. To substantially avoid this risk, the head portion of the otoscope according to the present disclosure (also having a tapered shape) preferably exhibits a diameter of at least 4mm, preferably of more than 5mm, more preferably of more than 6mm, at a position along the longitudinal axis of the head portion of no more than 4mm from a distal end point of the head portion. Thus, it is geometrically excluded to introduce the distal end of the head portion too far into the subject's ear canal. Different geometries of tapers may preferably be used according to the age group of the subject. For children, for example, the head portion of the otoscope adapted to carry out the method according to the present disclosure may exhibit a diameter of about 5mm at a position along the longitudinal axis of the head portion of no more than 4mm away from a distal end point of the head portion.

When introducing the tip end of the head portion no deeper into the ear canal than to the border between the outer part and the inner part of the outer ear canal of the patient's outer ear, there is the risk that artifacts, such as earwax, hair and other kind of dirt from the outer part of the outer ear canal obstruct the view of the small electronic imaging unit onto the patient's eardrum. Therefore, it is advantageous to take several images from different positions within the ear canal. For doing so, the otoscope according to the present disclosure may comprise more than one electronic imaging unit at the distal end of its head portion, e.g. two imaging units, located at different positions on the head portion.

The otoscope according to the present invention further comprises a motion mechanism configured to displace the electronic imaging unit relative to the handle portion. With such a motion mechanism, it is possible to capture a plurality of images from different positions by one single electronic imaging unit within the patient's ear canal, thereby avoiding the need for two or more electronic imaging units. If, for example, a hair - at least partially - obstructs the view of the electronic imaging unit at a certain position within the ear canal onto the eardrum, the electronic imaging unit may have a free view onto the eardrum at another position in the ear canal or may at least have a free view onto the part of the eardrum that was partially obstructed by the hair before.

Moreover, providing such a motion mechanism also allows for automatic identification of different objects in the patient's ear. Usually, in otoscopy, the eardrum represents the object of primary interest. In contrast, artifacts, such as earwax, hair and other kind of dirt, are usually of no particular interest. Such artifacts rather represent a problem when obstructing the view onto the patient's eardrum.

However, since artifacts are relatively close in front of the electronic imaging unit in the ear canal, compared to the eardrum, the artifacts can be distinguished from the eardrum when displacing the electronic imaging unit within the ear canal. That is, artifacts are depicted at distinct positions, if two images are captured from different positions/perspectives within the ear canal (due to their short distance to the electronic imaging unit), whereas the eardrum is shown substantially at the same position (due to the relatively large distance to the electronic imaging unit). According to the principle of stereoscopic viewing, the inventive device enables to determine the distance of different objects with respect to the electronic imaging unit. This determination can be automatically calculated by means of a logic unit, such as a microprocessor, preferably forming part of the otoscope. Furthermore, objects that have been identified as artifacts (due to their close distance to the electronic imaging unit) may be (automatically) eliminated by the image processing unit by comparing two or more images captured from different positions within the patient's ear canal. Consequently, a superimposed image may be generated or calculated by image processing means eliminating the artifacts. The image processing means may be implemented in form of a logic unit, such as a microprocessor provided in the otoscope. Thus, an image clearly depicting the eardrum can be obtained, even if the tip end of the head portion is introduced into the ear canal to the border between the outer part and the inner part of the outer ear canal (and not deeper into the ear canal).

The motion mechanism is preferably configured to allow at least partial rotation of the electronic imaging unit about an axis of rotation. The axis of rotation may correspond to the longitudinal axis of the head portion. By displacing the electronic imaging unit along a predefined motion path, it is possible to automatically calculate the distance of the electronic imaging unit to the detected objects, as described above. In view of the typical size of the artifacts found in the ear canal, such as hair and earwax particles, the motion mechanism preferably allows for displacement of the electronic imaging unit of at least 1 mm within the patient's ear canal. A rotation of at least 90°, more preferably of at least 120°, even more preferably of 180° or even more degrees around the axis may be realized. Preferably, the motion mechanism allows for rotation in both directions, i.e. clockwise and counter-clockwise. The motion mechanism may also allow for rotational displacement about more than one axis. The motion mechanism may comprise at least one motor and one or more gears and/or bearings. The electronic imaging unit may be connected to a flexible cable, e.g. a flexible ribbon cable, to allow for such a movement.

Preferably, the electronic imaging unit is tilted against the axis of rotation so as to be continuously directed to a predetermined point on the axis of rotation, the predetermined point having a fixed distance to the electronic imaging unit. In view of the typical length of the inner part of the outer ear canal of the patient's outer ear, the distance may be between 3mm and 20mm. Thus, the optical axis ("viewing direction") of the electronic imaging unit is optimized for centering on the eardrum, which usually represents the object of primary interest within the patient's ear.

For hygienic reasons, the otoscope preferably further comprises an at least partially transparent probe cover configured to be put over the head portion. The probe cover may be made from a plastic material, preferably from a transparent plastic material. Such a probe cover may be designed as a single-use product that can be produced in larger numbers with low costs. The probe cover shall be transparent, at least at the locations where it covers the electronic imaging unit, so as to allow the electronic imaging unit to have a clear view onto the eardrum. The probe cover also inhibits contamination of the head portion of the otoscope comprising the electronic imaging unit, in particular when introducing the head portion into the patient's ear canal.

Preferably, the probe cover is adapted to be fixed to at least one section of either the head portion and/or the handle portion in such a way that the probe cover does not move relative to the handle portion during displacement of the electronic imaging unit by the motion mechanism. Otherwise, artifacts, such as earwax particles, adhering to the probe cover will depicted by the electronic imaging unit, even if the electronic imaging unit is displaced by the motion mechanism. This, however, would interfere with object identification and elimination of artifacts from the captured images.

The otoscope may further comprise a probe cover moving mechanism adapted to move at least a portion of the probe cover with respect to the electronic imaging unit. Thus, artifacts, such as earwax particles, adhering to the probe cover and obstructing the view of the electronic imaging unit onto the eardrum can be moved away from the electronic imaging unit by the probe cover moving mechanism.

Preferably, the probe cover is designed in a way that allows unfolding or peeling of portions of the probe cover in order to move portions of the probe cover contaminated e.g. with earwax away from the electronic imaging unit. The otoscope preferably contains mechanical means to move the probe cover against the electronic imaging unit or vice versa.

To illuminate the patient's ear canal and eardrum, the otoscope may further comprise at least one light source typically positioned at the distal end of the head portion. The term "light source" is understood to apply to any source emitting photons.

Since geometrical restrictions limit the space at the distal end of the head portion, the light source is preferably formed by the distal end of a light guide. For example, the light guide may exhibit a diameter of less than 1 mm, preferably of less than 0.5mm, more preferably of about 0.2mm. The light guide may be connected to an LED located remote from the distal end of the head portion. The light guide may be e.g. a nylon light guide, preferably having a diameter of only about 0.2mm to 1 mm. Alternatively, a light source may be formed e.g. by a small light emitting diode that is placed directly at the distal end of the head portion.

It is advantageous, if the otoscope comprises a plurality of light sources at the distal end of the head portion, preferably with each light source being separately controllable. By illuminating objects in the patient's ear canal from different positions, e.g. by sequentially switching on and off the individual light sources, it may also be envisaged to distinguish different objects in the ear, without necessarily having to displace the electronic imaging unit by a motion mechanism within the ear canal. An object relatively far away from the electronic imaging unit, such as the eardrum, will change its appearance only slightly when being illuminated from different positions at the distal end of the head portion. However, artifacts that are relatively close to the electronic imaging unit (such as hair and earwax) will change their appearance (position) drastically. The otoscope therefore preferably comprises means, in particular a logic unit, such as a microprocessor, adapted to distinguish different objects in the patient's ear based on images taken with the objects being illuminated from different positions.

Additionally or alternatively, the at least one light source may be controllable in view of the color, so that it is possible to change the color of the light emitted by the light source. For example red color may be preferred to recognize an inflamed eardrum, wherein green color may be preferred to recognize earwax.

Like the electronic imaging unit, the at least one light source is preferably positioned radially offset from the longitudinal axis of the head portion. Such a configuration allows illumination of the eardrum without the need to introduce the light source as deeply into the ear canal as it would be necessary, if the light source were placed centrally on the longitudinal axis of the head portion. The offset may be at least 1 mm, preferably at least 2mm, more preferably at least 3mm from the longitudinal axis.

Preferably, the at least one light source is arranged so as to maintain a predetermined distance with respect to the electronic imaging unit, even when the electronic imaging unit is displaced by the motion mechanism. Such a configuration is advantageous, because the predetermined distal relationship between the at least one light source and the electronic imaging unit allows for improved (automatic) image analysis. If a motion mechanism is provided, the motion mechanism preferably also displaces the at least one light source. If the light source is provided in the form of a light guide, the light guide should be sufficiently flexible to allow for such a displacement of the at least one light source.

The otoscope may further comprise a logic unit, such as a microprocessor. The logic unit may be adapted to control the electronic imaging unit and/or the at least one light source. The logic unit may analyze the images obtained by the electronic imaging unit e.g. in order to detect an inflammation of the eardrum and/or the inner part of the outer ear canal, and/or in order to compare two images obtained with the electronic imaging unit located at different positions within the ear and/or with the object illuminated from different positions, so as to identify and discriminate different objects in the patient's ear. The logic unit may further be adapted to generate or calculate a new image wherein predetermined objects that have been previously identified are eliminated.

An exemplary embodiment of the present invention will be described in more detail in the following with respect to the drawings, wherein:
- figure 1: schematically shows a cross-sectional view of a head portion and of a part of a handle portion of an embodiment of an otoscope according to the present invention;
- figure 2: shows an enlarged view of a plate covering a bore provided in the head portion illustrated in figure 1; and
- figure 3: shows an otoscope of the prior art, with its head portion partially introduced into the patient's ear canal.

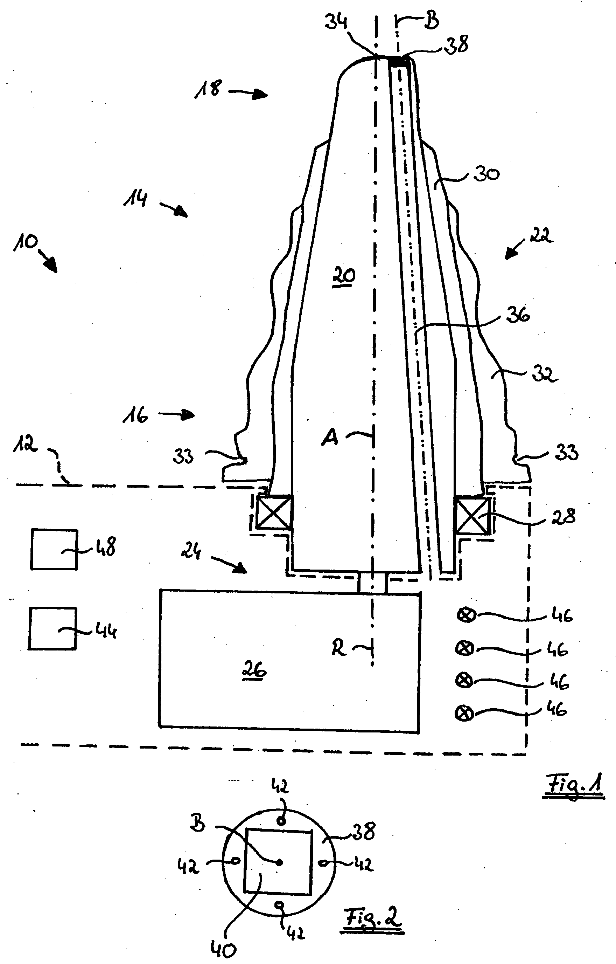

Figure 1 schematically shows a cross-sectional view of a head portion 14 and a part of a handle portion 12 (only shown in phantom lines) of an embodiment of an otoscope 10 according to the present invention. As can be seen from figure 1, the head portion 14 has a substantially tapering form extending along a longitudinal axis A of the head portion 14. The head portion 14 comprises a relatively large proximal end 16 adjacent to the handle portion 12 and a smaller distal end 18. The distal end 18 of the head portion 14 is adapted to be introduced into a patient's ear canal.

Furthermore, the head portion 14 comprises a rotatable, radial inner portion 20 and a fixed, radial exterior portion 22. The rotatable portion 20 is rotatable about an axis of rotation R which - in the shown exemplary embodiment - corresponds to the longitudinal axis A of the head portion 14. A motion mechanism 24 comprising a servo motor 26 is positioned within the handle portion 12 and is coupled to the rotatable portion 20 of the head portion 14, so as to rotate the rotatable portion 20 about its axis of rotation R relative to the fixed portion 22 of the head portion and relative to the handle portion 12 of the otoscope 10. The rotatable portion 20 is supported by a radial bearing 28 (also only schematically shown).

In the shown exemplary embodiment, the exterior portion 22 of the head portion 14 comprises a support structure 30 providing the required stability to the head portion 14. The support structure is at least partially covered by an outer cladding 32 formed from a relatively soft material, such as silicone. The cladding 32 makes it more comfortable for the patient to introduce the distal end 18 of the head portion 14 into his ear canal. The cladding may comprise a circular slot-like recess 33 adapted to engage with a complementarily formed circular tongue of a (not shown) probe cover. The probe cover may be formed from a plastic material and may be adapted to be put over the head portion 14. Preferably, the probe cover is formed from a transparent material. Its wall may be relatively thin, thereby making the probe cover relatively flexible. At least a portion of the probe cover covering the distal end 18 of the head portion 14 should be transparent, so as to allow an electronic imaging unit (described in the following) which is located at the distal end 18 of the head portion 14 to have a free view through the probe cover. For hygienic reasons, the probe cover is preferably designed as a single-use product. The probe cover also reliably inhibits contamination of the distal end 18 comprising the electronic imaging unit. Without such a probe cover there is a high risk that e.g. earwax particles may adhere to the electronic imaging unit (thereby deteriorating the image quality thereof) when introducing the distal end 18 into the outer part of the outer ear canal of the patient.

The head portion 14 comprises a distal end point 34 which, in the shown exemplary embodiment, is located substantially on the longitudinal axis A of the head portion 14. However, the head portion 14 might alternatively have a tapering shape that is not substantially symmetrical to its longitudinal axis A (as shown in figure 1) but is more adapted to the anatomy of the human ear canal.

Irrespective of the precise shape of the head portion 14, the head portion 14 is preferably dimensioned in such a way that it cannot be introduced into the inner part of the outer ear canal of the patient's outer ear. In the shown exemplary embodiment, the distal end 18 of the head portion 14 has a substantially round shape. Only a few millimeters (less than 4mm) away from the distal end point 34 in the direction of the longitudinal axis A, the head portion 14 exhibits a diameter of more than 5mm. Since the inner part of the outer ear canal of an adult usually exhibits a diameter of 4mm, there is no risk that the distal end 18 of the head portion 14 is inadvertently introduced too deeply into the patient's ear canal. Therefore, injuries to the sensitive skin of the inner part of the outer ear canal and/or to the eardrum can be reliably avoided.

The movable portion 20 comprises a bore 36 extending substantially along the axial direction A of the head portion 14, but not exactly parallel thereto. The distal end of the bore 36 is located in proximity to the distal end point 34, but offset with its bore axis B by at least 2mm from the longitudinal axis A. Furthermore, the distal end of the bore 36 is closed by a plate 38. An enlarged top view of the plate 38 is shown in figure 2. Since the bore 36 is cylindrical in shape, the plate 38 has a generally circular appearance in figure 2 with the bore axis B forming the center thereof. However, the bore 30 and/or the plate 38 may equally exhibit other shapes.

The plate 38 supports a wide-angle color video camera 40 and distal ends of four light guides 42. In the exemplary embodiment, the light guides 42 are located around the video camera 40, such that one light guide 42 is associated to each of the four lateral sides of the substantially rectangular video camera 40. However, this is not a prerequisite for the present invention. Instead of four light guides 42, for example, only two light guides 42 may be provided in the otoscope 10. The video camera 40 is advantageously a wafer-level camera of dimensions in the 1 to 2mm range having a substantially flat configuration. The wafer-level camera advantageously exhibits dimensions of only about 1 mm x 1 mm providing a resolution of about 250 pixels of 250 pixels. The plate 38 has a diameter between 1.5mm and 2.0mm and the light guides 42 have a diameter of only about 0.2mm.

The video camera 40 is connected to a distal end of a cable (not shown). The cable, e.g. a ribbon cable, extends through the bore 36 and into the handle portion 12 of the otoscope 10. A distal end of the cable is connected to a logic unit 44, such as a microprocessor, which is schematically illustrated in figure 1. Similarly, the light guides 42 (not shown in figure 1) extend through the bore 36 and into the handle portion 12 of the otoscope 10. Proximal ends of the light guides 42 are connected to four LEDs 46, respectively. The LEDs 46 are positioned - like the logic unit 44 - within the handle portion 12 of the otoscope 10. The LEDs 46 can be individually switched on and off. Furthermore, the handle portion 12 preferably comprises a memory 48 for storing images captured by the video camera 40. The memory may be formed e.g. by a storage card slot and a corresponding storage card inserted in the slot. The handle portion 12 may further comprise a display (not shown) for displaying the images taken by the camera 40 to the user. Additionally or alternatively, the handle portion 12 may comprise a cable connection port, such as an USB-port, and/or a wireless connection, such as Bluetooth®, WIFI® and/or an energy supply, such as a (rechargeable) battery. These additional (optional) components of the handle portion 12 are known e.g. from digital cameras.

For capturing images of a patient's inner part of the outer ear canal, and in particular of a patient's eardrum, the distal end 18 of the head portion 14 has to be introduced into the patient's ear canal. Due to the shape of the head portion 14 there is no risk to insert the distal end 18 too deeply into the ear canal. That is, the shape and geometry of the distal end If does not allow significantly introducing the distal end point 34 into the patient's inner part of the outer ear canal which is pain sensitive. Therefore, injuries to the skin of the inner part of the outer ear canal and/or the eardrum can be reliably avoided. The geometry and the technology of the disclosed otoscope do not require deforming the patient's ear as with a classic otoscope, as described above. Consequently, the otoscope according to the present disclosure can also be securely applied by laypersons.

Even though the distal end 18 of the head portion 14 will not be inserted into the inner part of the outer ear canal, the otoscope according to the present disclosure, nevertheless, allows for capturing images from the inner part of the outer ear canal and the eardrum, because of the wide angle camera 40 being provided at the distal end 18 of the head portion 14. In order to improve the ability of the camera 40 to "see" the eardrum, the camera 40 is placed offset from the longitudinal axis A of the head portion 14. Furthermore, the main "viewing direction" of the camera 40, corresponding to the bore axis B, is angled with respect to the longitudinal axis A of the head portion 14. The bore axis B and the longitudinal axis A intersect at a point having a predetermined distance from the distal end point 34, wherein the predetermined distance corresponds to the typical length of a patient's inner part of the outer ear canal, so that the camera 40 is directed to the eardrum.

When the distal end 18 of the head portion is introduced in the patient's ear canal, it may happen that artifacts, such as earwax particles or hair, in front of the camera 40, e.g. adhering to the probe cover, partially or even fully obstruct the view onto to eardrum. Therefore, the motion mechanism 24 may turn the rotatable portion 20 of the head portion 14 with respect to the remaining otoscope 10 about its axis of rotation R. For example, the motion mechanism 24 may rotate the rotatable portion 20 from an initial position by about 120° in clockwise direction, then from the initial position by about 120 in counter-clockwise direction, and finally return to the initial position. The camera 40 may capture one or more images from each of these equally spaced three positions. The logic unit 44 may identify different objects in the patient's ear by comparing the images received from the camera 40. In particular, the logic unit 44 may discriminate artifacts from the eardrum by determining their distance to the camera 40 according to the principle of stereoscopic viewing, as described in more detail above.

In order to further improve the identification process more than one image may preferably be taken from each of the three positions of the camera 40, with different LEDs 46 switched on and off for each captured image. Illumination of the artifacts and the eardrum from different positions also assists to discriminate these objects, as described in more detail above.

Finally, a new image may be generated (preferably by the logic unit 44) in which the identified artifacts are eliminated, so as to clearly show the eardrum. The degree of reddishness of the eardrum can then be easily determined. The user may be provided with corresponding information, such as to see the physician because of the risk of otitis media, or not. Also if the otoscope failed to detect the eardrum because of massive earwax in the patient's ear canal, corresponding information may be provided to the user. The user may then decide to visit a physician for having his or her ear canal cleaned.

## Claims

1. Otoscope (10) comprising:
- a handle portion (12) allowing a user to manipulate the otoscope (10) during its application; and
- a head portion (14) exhibiting a substantially tapering form extending along a longitudinal axis (A) of the head portion (1-4), wherein the head portion (14) has a proximal end (16) adjacent to the handle portion (12) and a smaller distal end (18) configured to be introduced in an ear canal of a patient's outer ear,
wherein the otoscope (10) further comprises an electronic imaging unit (40) positioned at the distal end (18) of the head portion (14), wherein the electronic imaging unit (40) is a camera,
**characterized in that**
the otoscope further comprises a motion mechanism (24) configured to displace the electronic imaging unit (40) relative to the handle portion (12).

2. Otoscope (10) according to claim 1, wherein the electronic imaging unit (40) is a video camera (40), preferably a wide angle color video camera (40).

3. Otoscope (10) according to claim 1 or 2, wherein the electronic imaging unit (40) is a wafer-level camera (40) of a substantially flat configuration, having dimensions of less than 3mm x 3mm, preferably less than 2mm x 2mm, even more preferable about 1 mm x 1 mm or less than 1 mm x 1 mm.

4. Otoscope (10) according to any one of the preceding claims, wherein the motion mechanism (24) is configured to allow at least partial rotation of the electronic imaging unit (40) about an axis of rotation (R), wherein the axis of rotation (R) preferably corresponds to the longitudinal axis (A) of the head portion (14).

5. Otoscope (10) according to claim 4, wherein the electronic imaging unit (40) is tilted against the axis of rotation (R) so as to be continuously directed to a predetermined point on the axis of rotation (R), the predetermined point having a fixed distance to the electronic imaging unit (40).

6. Otoscope (10) according to any one of the preceding claims, further comprising an at least partially transparent probe cover configured to be put over the head portion (14) wherein the probe cover is adapted to be fixed to at least one portion of the head portion (14) and the handle portion (12) in such a way that the probe cover does not move relative to the handle portion (12) during displacement of the electronic imaging unit (40) by the motion mechanism (24).

7. Otoscope (10) according to claim 6, further comprising a probe cover moving mechanism adapted to move at least a portion of the probe cover with respect to the electronic imaging unit (40).

8. Otoscope (10) according to any one of the preceding claims, further comprising at least one light source also positioned at the distal end (18) of the head portion (14)
wherein the at least one light source is arranged so as to maintain a predetermined distance with respect to the electronic imaging unit (40), even when the electronic imaging unit (40) is displaced by the motion mechanism (24).

## Patentansprüche

1. Otoskop (10), umfassend:
einen Griffabschnitt (12), der einem Benutzer die Manipulation des Otoskops (10) bei einer Anwendung ermöglicht; und einen Kopfabschnitt (14) der eine sich entlang einer Längsachse (A) des Kopfabschnitts (14) im Wesentlichen verjüngende Form aufweist,
wobei der Kopfabschnitt (14) ein proximales Ende (16), das an den Griffabschnitt (12) angrenzt, und ein kleineres distales Ende (18) aufweist, das dazu konfiguriert ist, in einen Ohrkanal des äußeren Ohrs eines Patienten eingeführt zu werden, wobei das Otoskop (10) ferner eine elektronische Bilderfassungseinheit (40) am distalen Ende (18) des Kopfabschnitts (14) beinhaltet, wobei die elektronische Bilderfassungseinheit (40) eine Kamera ist,
**dadurch gekennzeichnet,**
**dass** das Otoskop ferner einen Bewegungsmechanismus (24) beinhaltet, der dazu konfiguriert ist, die elektronische Bilderfassungseinheit (40) relativ zu dem Griffabschnitt (12) zu versetzen.

2. Otoskop (10) nach Anspruch 1, wobei die elektronische Bilderfassungseinheit (40) eine Videokamera (40), vorzugsweise eine Weitwinkel-Farbvideokamera (40) ist.

3. Otoskop (10) nach Anspruch 1 oder 2, wobei die elektronische Bilderfassungseinheit (40) eine Wafer-Level-Kamera (40) mit einer im wesentlichen flachen Konfiguration und mit Abmessungen von weniger als 3 mm x 3 mm ist, vorzugsweise weniger als 2 mm x 2 mm , noch bevorzugter etwa 1 mm x 1 mm oder weniger als 1 mm x 1 mm.

4. Otoskop (10) nach einem der vorhergehenden Ansprüche, wobei der Bewegungsmechanismus (24) dazu konfiguriert ist, eine zumindest teilweise Drehung der elektronischen Bilderfassungseinheit (40) um eine Drehachse (R) zu ermöglichen, wobei die Drehachse (R) vorzugsweise der Längsachse (A) des Kopfabschnitts (14) entspricht.

5. Otoskop (10) nach Anspruch 4, wobei die elektronische Bilderfassungseinheit (40) gegen die Rotationsachse (R) geneigt ist, um kontinuierlich zu einem vorgegebenen Punkt auf der Rotationsachse (R) geleitet zu werden, wobei der vorgegebene Punkt in einem festen Abstand zu der elektronischen Bilderfassungseinheit (40) liegt.

6. Otoskop (10) nach einem der vorhergehenden Ansprüche, weiterhin umfassend eine zumindest teilweise transparente Sondenabdeckung, die dazu konfiguriert ist, um über den Kopfabschnitt (14) gestülpt zu werden, wobei die Sondenabdeckung dazu angepasst ist, um zumindest an einem Abschnitt des Kopfabschnitts (14) und des Griffabschnitts (12) in einer Weise befestigt zu werden, dass die Sondenabdeckung sich nicht relativ zu dem Griffabschnitt (12) während der Verschiebung des elektronischen Bildverarbeitungseinheit (40) durch den Bewegungsmechanismus (24) bewegt.

7. Otoskop (10) nach Anspruch 6, ferner umfassend einen Sondenabdeckung-Bewegungsmechanismus, der dazu eingerichtet ist, wenigstens einen Teil der Sondenabdeckung in Bezug auf die elektronische Bilderfassungseinheit (40) zu bewegen.

8. Otoskop (10) nach einem der vorhergehenden Ansprüche, weiterhin beinhaltend mindestens eine Lichtquelle, die auch an dem distalen Ende (18) des Kopfabschnitts (14) positioniert ist, wobei die mindestens eine Lichtquelle so angeordnet ist, dass sie auch dann einen vorbestimmten Abstand in Bezug auf die elektronische Bilderfassungseinheit (40) beibehält, selbst wenn die elektronische Bilderzeugungseinheit (40) durch den Bewegungsmechanismus (24) verschoben wird.

## Revendications

1. Otoscope (10) comprenant :
- une partie manche (12) permettant à un utilisateur de manipuler l'otoscope (10) durant son application ; et
- une partie tête (14) présentant une forme sensiblement conique s'étendant le long d'un axe longitudinal (A) de la partie tête (14), dans lequel la partie tête (14) comporte une extrémité proximale (16) adjacente à la partie manche (12) et une extrémité distale (18) configurée pour être introduite dans un conduit auditif de l'oreille externe d'un patient,
dans lequel l'otoscope (10) comprend en outre une unité d'imagerie électronique (40) positionnée à l'extrémité distale (18) de la partie tête (14), dans lequel l'unité d'imagerie électronique (40) est une caméra,
**caractérisé en ce que**
l'otoscope comprend en outre un mécanisme de mouvement (24) configuré pour déplacer l'unité d'imagerie électronique (40) par rapport à la partie manche (12).

2. Otoscope (10) selon la revendication 1, dans lequel l'unité d'imagerie électronique (40) est une caméra vidéo (40), de préférence une caméra vidéo en couleur grand-angle (40).

3. Otoscope (10) selon la revendication 1 ou 2, dans lequel l'unité d'imagerie électronique (40) est une caméra sur tranche (40) d'une configuration sensiblement plate, ayant des dimensions de moins de 3 mm x 3 mm, de préférence de moins de 2 mm x 2 mm, encore plus préférable d'environ 1 mm x 1 mm ou de moins de 1 mm x 1 mm.

4. Otoscope (10) selon l'une quelconque des revendications précédentes, dans lequel le mécanisme de mouvement (24) est configuré pour permettre une rotation au moins partielle de l'unité d'imagerie électronique (40) autour d'un axe de rotation (R), dans lequel l'axe de rotation (R) correspond de préférence à l'axe longitudinal (A) de la partie tête (14).

5. Otoscope (10) selon la revendication 4, dans lequel l'unité d'imagerie électronique (40) est inclinée contre l'axe de rotation (R) de manière à être dirigée en continu vers un point prédéterminé sur l'axe de rotation (R), le point prédéterminé ayant une distance fixe par rapport à l'unité d'imagerie électronique (40).

6. Otoscope (10) selon l'une quelconque des revendications précédentes, comprenant en outre un protège-sonde au moins partiellement transparent configuré pour être posé par-dessus la partie tête (14) dans lequel le protège-sonde est adapté pour être fixé sur au moins une partie parmi la partie tête (14) et la partie manche (12) d'une manière telle que le protège-sonde ne se déplace pas par rapport à la partie manche (12) durant le déplacement de l'unité d'imagerie électronique (40) par le mécanisme de mouvement (24).

7. Otoscope (10) selon la revendication 6, comprenant en outre un mécanisme de mouvement de protège-sonde adapté pour déplacer au moins une partie du protège-sonde par rapport à l'unité d'imagerie électronique (40).

8. Otoscope (10) selon l'une quelconque des revendications précédentes, comprenant en outre au moins une source de lumière également positionnée à l'extrémité distale (18) de la partie tête (14)
dans lequel l'au moins une source de lumière est agencée de manière à maintenir une distance prédéterminée par rapport à l'unité d'imagerie électronique (40), même quand l'unité d'imagerie électronique (40) est déplacée par le mécanisme de mouvement (24).
